Europäisches Patentamt

(19) European Patent Office

Office européen des brevets

(11) Publication number: **0 328 531 B1**

(12) EUROPEAN PATENT SPECIFICATION

(45) Date of publication of patent specification :
05.06.91 Bulletin 91/23

(21) Application number : 87906594.4

(22) Date of filing : 13.10.87

(86) International application number :
PCT/GB87/00716

(87) International publication number :
WO 88/02636 21.04.88 Gazette 88/09

(51) Int. Cl.⁵ : **A61L 15/07**

(54) **BANDAGES.**

(30) Priority : 14.10.86 GB 8624645

(43) Date of publication of application :
23.08.89 Bulletin 89/34

(45) Publication of the grant of the patent :
05.06.91 Bulletin 91/23

(84) Designated Contracting States :
AT BE CH DE FR GB IT LI LU NL SE

(56) References cited :
EP-A- 0 086 621
FR-A- 1 449 774

(73) Proprietor : SMITH & NEPHEW plc
2 Temple Place Victoria Embankment
London WC2R 3BP (GB)

(72) Inventor : HICKS, John, Kenneth
92 Boulevard
Hull, North Humberside (GB)
Inventor : FRY, Nicholas, Charlton
572 Hotham Road South
Hull, North Humberside (GB)

(74) Representative : Cole, William Gwyn, Dr.
Smith and Nephew Research Limited Gilston
Park
Harlow Essex CM20 2RQ (GB)

## Description

The present invention relates to a water hardenable, polyurethane orthopaedic splinting bandage comprising a flexible fabric supporting a water curable isocyanate terminated prepolymer, a catalyst for the curing reaction and additive which stabilises the splinting bandage against premature setting of the bandage prior to use. More particularly the invention relates to bandages in which the additive is an organic acid anhydride. The present invention also relates to a method of making a splinting bandage, to water curable prepolymers and water hardenable compositions containing the organic acid anhydride and to processes for their preparation.

In recent years attempts have been made to make water hardenable or curable orthopaedic bandages from polymeric materials such as polyurethane and polyacrylate. Conventionally such bandages are formed from an air permeable, water permeable flexible fabric impregnated and/or coated with a reactive prepolymer system which is hardenable on exposure to water usually in the presence of a catalyst. In one type of known system the prepolymer is an isocyanate terminated prepolymer formed by the reaction of an isocyanate and a polyol. This prepolymer hardens in the presence of water and a catalyst. One problem experienced by bandages incorporating this type of prepolymer and in particular a prepolymer derived from polyethylene glycol is the somewhat short shelf life. This can be caused by the bandages being exposed to moisture or side reactions such as allophonate formation within the prepolymer in storage prior to use, whereupon the bandages will slowly harden and become useless. It is believed this can occur to such bandages in spite of carefully controlled manufacturing processes and packing in barrier packaging. It is known in the art that the storage stability of a polyurethane splinting bandage can be improved by adding a stabiliser to the bandage prepolymer to inhibit premature curing. United States Patent Nos. 4433680 and 4655208 disclose polyurethane splinting bandages comprising prepolymers stabilised with benzoyl chloride. It has been found, however, that benzoyl chloride is highly lachcrymatory and will give the bandage an unpleasant smell. United States Patent No. 4574793 discloses polyurethane splinting bandages comprising prepolymers stabilised with methane sulphonic acid. Methane sulphonic acid, however, is a strong acid which, if mixed therewith, would breakdown the glycol components of a reaction mixture used to form a polyurethane prepolymer. Such a stabiliser therefore cannot be added to the reaction mixture to stabilise the prepolymer during its preparation. Polyurethane splinting bandages with good resistance to premature setting have now been discovered which comprise a prepolymer in admixture with a stabiliser which avoids the disadvantages of the prior art stabilisers hereinabove discussed.

Accordingly the present invention comprises a water hardenable polyurethane splinting bandage comprising a flexible fabric carrying a water curable isocyanate terminated prepolymer and a catalyst therefor wherein the prepolymer is in admixture with an organic acid anhydride.

The organic acid anhydride stabilises the prepolymer thereby inhibiting the premature setting of the bandage. It is believed that the organic acid anhydride stabilises the prepolymer by reacting with any residual or absorbed moisture or aqueous alkali component in the prepolymer which promotes the curing thereof and by forming in the presence of such components, an acidic environment which inhibits the prepolymer from curing prematurely.

Apt organic acid anhydrides are hydrocarbyl anhydrides in which the hydrocarbyl suitably contains 1 to 12 carbon atoms preferably 2 to 6 carbon atoms and may be those derived from aliphatic, alicyclic or aromatic acids which form anhydrides. Suitable anhydrides are preferably cyclic anhydrides. Favoured organic acid anhydrides include those derived from polycarboxylic acids such as aliphatic, alicyclic aromatic dibasic acids. A preferred organic acid anhydride is succinic anhydride. Such a preferred anhydride is odourless and will not discolour the product. Less favoured organic acid anhydrides are those derived from aromatic dibasic acid such as phthallic anhydride.

Suitably the organic acid anhydride will be present as a solid in the dry prepolymer, that is the anhydride will be insoluble in the prepolymer. Suitably the organic acid anhydride will be in finely divided form that is the particles will suitably have a diameter of less than 150 μm, and favourably below 100 μm. The average particle size of the anhydride is generally below 75 μm and is preferably from 30 to 40 μm.

Suitably the organic acid anhydride will be present in an amount of 0.1 to 5% by weight of the prepolymer, for example 0.78%, 1.0% and 1.25%. Other stabilisers may be present in the prepolymer in addition to the organic acid anhydride. An apt stabiliser is methane sulphonic acid which may be present in an amount which is 0.01 to 1.0% by weight of the prepolymer.

Suitable isocyanate terminated prepolymers for use in the splinting bandages of the present invention include those described in our United Kingdom Patent No. 2092606B at page 2 lines 12 to 21 and page 4 line 13 to page 10 line 13 and in our United States Patent No. 4427003.

Other suitable isocyanate terminated prepolymers for use in the splinting bandages of the present invention include those described in Germany Offenlegungschriften Nos. 2353212 and 2357931, and European Patent No. 0086621.

2

Highly suitable water curable prepolymers for use in the invention are derived from a polyol and an isocyanate and also contain an organic acid anhydride stabiliser.

In another aspect the invention provides a water curable isocyanate terminated prepolymer for use in a bandage of the invention in which the prepolymer is derived from a polyol and an isocyanate and contains an organic acid anhydride.

Highly suitable prepolymers for use in the splinting bandage of the present invention are derived from polyols which are polyoxyalkylene oxide adducts such as ethylene oxide adducts, propylene oxide adducts, tetramethylene oxide adducts or mixtures thereof. Favoured prepolymers for use in the invention however comprise a polyol derived from an ethylene oxide adduct.

The polyol will normally have a reaction functionality of two or more than two and an isocyanate which has a reaction functionality of two or more than two. The reaction functionality of the isocyanate is preferably between two and three and is most preferably two.

A preferred isocyanate terminated prepolymer is derived from an aromatic isocyanate having a reaction functionality of between 2 and 3 and a polyol mixture containing at least one diol which is preferably a polyoxyethylene diol and at least one triol.

The polyol mixture will aptly contain sufficient oxyethylene units to render the prepolymer water absorbent. Such water absorbent prepolymers advantageously lower the set times and also the surface tack of a setting bandage.

In a further aspect the present invention provides a water hardenable composition for use in a bandage of the invention which comprises an isocyanate terminated prepolymer which contains an organic acid anhydride of the invention and a catalyst.

Suitable catalysts for use in the water hardenable splinting bandages of the present invention include those described in United Kingdom Patent No. 2092606B at page 3 line 10 to page 4 line 11 and in United States Patent No. 4427003. Such catalysts are water soluble but insoluble in the prepolymer. Aptly the catalyst is an inorganic material which has an alkaline reaction in water. Suitably the catalyst is a solid water soluble carbonate or bicarbonate and includes alkali metal carbonates and bicarbonates. Preferred catalysts are sodium carbonate and potassium carbonate of which potassium carbonate is preferred.

Other catalysts which may be employed in the water hardenable splinting bandage of the present invention include these described in United Kingdom Patent No. 1578895 which employs amino polyols as catalysts and as the polyol components, those disclosed in International Application No. WO 81/00671 which employs dimethyl ethanolamine or a mixture of dimethyl ethanolamine and bis(2-dimethylaminoethyl) ether and those disclosed in European Patent No. 0086621 and United States Patent No 4574793 including dimorpholino-diethylether and bis(2,6-dimethylmorpholino) diethylether catalysts.

The amount of catalyst present in admixture with the prepolymers of the present invention will normally be from 0.1 to 40% by weight. Suitably the amount of alkali metal carbonate or bicarbonate catalyst will be 2 to 20% by weight and preferably will be 3 to 10% by weight of the prepolymer, for example 4,5 or 6%. Suitably the amount of dimorpholino-diethylether or bis(2,6-dimethylmorpholino) diethylether catalyst will be 0.1 to 10% of weight and preferably 0.3 to 4% by weight of the prepolymer.

Favoured flexible fabrics for carrying the prepolymer, catalyst and anhydride in the bandages of the present invention include those described in United Kingdom Patent No. 2092606B at page 10 lines 14 to page 11 line 10 and United States Patent No. 4427003 and in our copending European Patent Application No. 94222 at pages 38 and 39.

The bandages may be conventionally packaged in heat sealed polyethylene pouches such as metal foil polyethylene laminate pouches. Although such pouches are meant to exclude water and moisture vapour, it has been found difficult to ensure teat moisture vapour is excluded from the pouches on prolonged storage and so in some instances the water-curable bandages will prematurely set. It has been found that with bandages in which the water-curable prepolymer includes an organic acid anhydride the tendency for premature setting is much reduced so giving the bandages of the invention a longer storage capability than has hitherto been available without such a stabiliser.

In use the bandages are brought into contact with water normally by immersion and are wrapped around an injured limb. The setting bandage has a working time sufficient to allow the bandage to be positioned on the affected part of the body and a set time at which the cast is rigid. The presence of the organic acid anhydride in the prepolymer allows the bandage to possess working and set times which are suitably 1 to 6 minutes and 5 to 30 minutes respectively.

The prepolymers may be prepared by the conventional methods which are described in United Kingdom Patent No. 2092606. The organic acid anhydride can be mixed with the prepolymer after preparation. It has been found, however, that prepolymers prepared using a polyethylene glycol component tend to set prematurely during or immediately after preparation. It is therefore preferred, when such a prepolymer is prepared, to mix the organic acid with the polyethylene glycol component prior to preparation of the prepolymer to inhibit setting of the prepolymer in the reaction container.

Thus in another aspect the invention provides a process for preparing a water curable isocyanate terminated prepolymer of the invention which comprises mixing a polyol with an organic acid anhydride and reacting the polyol with excess isocyanate.

The prepolymer can then be mixed with a catalyst to prepare a water curable hardenable composition of the invention.

Thus in a further aspect the invention provides a process for preparing a water hardenable composition of the invention which comprises mixing a catalyst with an isocyanate terminated prepolymer which contains an organic acid anhydride of the invention.

The catalyst which may be a particulate solid water soluble catalyst such as potassium carbonate or a liquid catalyst such as dimorpholino-diethylether or bis(2,6-dimethylmorpholino) diethylether may be mixed into the prepolymer by a conventional method.

Inert materials may be incorporated into the prepolymer mixture, if necessary, as fillers, reinforcing fillers or colouring agents.

The prepolymer mixture can then be used to prepare a bandage of the invention.

In another aspect the invention provides a method of making a water hardenable polyurethane splinting bandage of the invention which comprises coating or impregnating a flexible fabric carrier, which has apertures of sufficient size to allow water to permeate the bandage and react with the prepolymer, with a mixture of a water curable isocyanate terminated prepolymer containing an organic acid anhydride inhibitor and a catalyst.

The flexible fabric may be coated or impregnated and made into bandages by conventional methods known in the art.

The invention will be now illustrated by references to the following examples.

## EXAMPLE 1

### Preparation of Prepolymer

The prepolymer was prepared from the following components :

```
Polyethylene glycol (mol wt 1000)        1 moles

Polypropylene glycol (mol wt 1025)    1.25 moles

Methylene-bis-(4 cyclohexyl-

    isocyanate) sufficient to give

    a mole ratio of NCO/OH of            3.75/1

Succinic anhydride                       1%  based on

                                         weight of

                                         components.
```

The glycols were mixed together with the finely ground succinic anhydride in a reactor which was adapted to be purged with dry nitrogen. The isocyanate was added and the reactor purged with dry nitrogen. The reaction mixture was then stirred for 90 minutes at 55°C. The prepolymer formed was allowed to cool and then discharged into a container which had previously been purged with dry nitrogen. The container was sealed until the prepolymer was required.

### Preparation of Bandage

A portion of the prepolymer (100 g) prepared above was mixed with Analar grade potassium carbonate (6 g) and 0.2% antifoaming agent. This prepolymer mixture was spread onto 8 cm wide 612 cotton leno gauze by a blade over flat bed coating technique as a hot melt at a temperature of approximately 60°C cotton in a closed area purged with carbon dioxide to obtain a dry atmosphere. The bandage strip had an apertured coating of prepolymer at a weight per unit area of 62 gsm. The bandage strip was cut into 1 metre lengths and rolled up. The bandage rolls were then stored in heat sealed pouches of 62.5 µm thick low density polyethylene which had previously been purged with nitrogen. Prepolymers containing succinic anhydride showed satisfactory ageing characteristics.

The bandage may be made into a cast by immersing the bandage in water and wrapping the unrolled bandage around a 2.5 cm diameter spindle. The bandage will have working time of 5 minutes and a set time of 12 minutes. The resulting cast will be rubbery at first but will rapidly harden.

## EXAMPLE 2

The prepolymer is prepared from the following components :

| | |
|---|---|
| Poly (oxypropylene) glycol (mol wt 1025) | 44.0g |
| Poly (oxypropylene) triol (mol wt 400) | 31.0g |
| Modified diphenylmethane diisocyanate (Isonate 143-L) | 12.0g |
| Dimorpholino-diethyl ether | 3.4g |
| Succinic anhydride | 1% based on weight of components |

The isocyanate is placed in a reactor which is purged with nitrogen. The glycols are mixed together, dried and mixed with the finely ground succinic anhydride. The polyol mixture is added to the reactor using a dropping funnel over a period of 25 to 30 minutes. After the addition is complete, the reaction mixture is then stirred for 1 hour at 55°C. The prepolymer formed is allowed to cool, mixed with dimorpholino-diethylether and then discharged into a container which has been previously purged with dry nitrogen. The container is sealed until the prepolymer is required.

Bandages may be prepared by the method described in Example 1.

## EXAMPLE 3

The prepolymer is prepared from the following components :

| | |
|---|---|
| Poly (oxypropylene) diol (mol wt 1025) | 80g |
| Modified diphenyl methane diisocyanate (Isonate 143-L) | 120g |
| N,N-dimethylethanolamine | 2ml |
| Succinic anhydride | 1% based on weight of components |

The isocyanate is placed in a reactor which is fitted with a stirrer, protected from ingress of moisture and may be purged with dry nitrogen. Under an atmosphere of dry nitrogen a mixture of the diol, amine and finely divided succinic anhydride is added slowly to the stirred isocyanate. The temperature of the reaction mixture is kept below 50°C. The prepolymer formed is allowed to cool and is then discharged into a dry sealable container which has been previously purged with dry nitrogen.

Bandages may be prepared by the method described in Example 1.

## EXAMPLE 4

The prepolymer is prepared from the following components :

```
Diphenylmethane diisocyanate        50g

Propoxylated triethanolamine        40g

Succinic anhydride                  1.2% based

                                    on the

                                    weight of

                                    components
```

The prepolymer is prepared in a similar manner to that described in Example 3.

An analogous prepolymer is prepared using phthallic anhydride.

## EXAMPLE 5

A prepolymer was formed from the following components :

```
PEG    1000                         3 moles

Poly (oxytetramethylene) glycol

        (Mol wt. 1000)              3 moles

Arcol 3130                          4 moles

Isonate 143 - L                     sufficient to give

                                    a NCO to OH ratio

                                    of 3.53 : 1

Succinic anhydride                  1% by weight of the

                                    components
```

The prepolymer of this example was prepared in the same manner as example 1, the finely ground succinic anhydride being mixed with the PEG 1000 prior to being mixed with the other polyols. PEG 1000 is a polyoxyethylene glycol with an average molecular weight of approximately 1000 available from B.P. Chemicals.

Arcol 3130 is a polyoxypropylene triol with an average molecular weight of approximately 400 available from Arco Chemicals.

Isonate 143 – L is a diphenylmethane – 4,4'-diisocyanate containing polycarboniimide adducts available from Dow Chemicals.

A bandage, using the prepolymer prepared above, was prepared in the same manner as Example 1 except that the amount of potassium carbonate mixed with the prepolymer was 3% by wt. and the prepolymer mixture was coated by means of nip rollers at 22°C on to a flexible knitted glass fibre substrate (length 350 cm, width 7.5 cm) at a weight per unit area of 25 g/m$^2$.

The bandage rolls were stored in individual heat sealed polyethylene coated aluminium foil pouches, which had been previously purged with nitrogen.

## EXAMPLE 6

A prepolymer was prepared in the same manner as Example 5 using the following components :

```
P E G   1000                            6 moles

Arcol 3150                              4 moles

Isonate 143 - L           sufficient to give a

                          NCO to OH ratio of

                          3.53 : 1

Succinic anhydride        1% by weight of the

                          components.
```

Analogous prepolymers were prepared using phthallic anhydride.
Bandages containing the prepolymers were prepared and stored in the same manner as Example 5.

## EXAMPLE 7

A prepolymer was prepared in the same manner as Example 5 using the following components :

```
P E G   1000                            3 moles

PPG     1025                            3 moles

Arcol   3150                            4 moles

Isonate 143 - L           sufficient to give a

                          NCO to OH ratio of

                          3.53 : 1

Succinic anhydride        1% by weight of

                          components.
```

PPG 1025 is a polyoxypropylene glycol having an average molecular weight of approximately 1025, available from B.P. Chemicals.

Bandages containing the prepolymer mixed with 6% by weight of potassium carbonate were prepared and stored in the same manner as Example 5.

## EXAMPLE 8

A prepolymer was prepared from the following ingredients :

7

| P E G    1000 | 6 moles |
| Arcol    3150 | 4 moles |
| Isonate 143 - L | sufficient to give a NCO to OH ratio of 3.53 : 1 |
| Succinic anhydride | 1% be weight |
| Methane Sulphonic Acid | 0.03% of components |
| Antifoam | 0.3% by weight. |

The prepolymer was prepared in the same manner as Example 5, the antifoam being mixed with the polyol components and the methane sulphonic acid being added to the prepolymer after it had been prepared.

Bandages, containing the prepolymer, were prepared and stored in the same manner as Example 5 except that the prepolymer contained 0.55% by weight of catalyst bis(2,6-dimethylmorpholino) diethylether instead of potassium carbonate.

The bandages of Examples 5 to 8 on initial storage did not exhibit premature setting. In addition the bandages of Example 7 were found to be satisfactory after being stored for 6 months under accelerated ageing conditions at a temperature of 40°C.

The bandages of these samples before and after storage were found to have satisfactory working and set times when used to form a cast.

## Claims

1. A water hardenable polyurethane splinting bandage comprising a flexible fabric carrying a water curable isocyanate terminated prepolymer and a catalyst therefor characterised in that the prepolymer is in admixture with an organic acid anhydride.

2. A bandage as claimed in claim 1 which the anhydride is an aliphatic dibasic acid anhydride.

3. A bandage as claimed in claim 2 in which the anhydride is succinic anhydride.

4. A bandage as claimed in any of claims 1 to 3 in which the anhydride is in particulate form and is present as 0.5% to 1.5% by weight of the prepolymer.

5. A water curable isocyanate terminated prepolymer for use in a bandage of any claims 1 to 4 in which the prepolymer is derived from a polyol and an isocyanate and contains an organic acid anhydride.

6. A prepolymer as claimed in claim 5 in which the anhydride is succinic anhydride.

7. A prepolymer as claimed in either claims 5 or 6 in which polyol is an ethylene oxide adduct and the isocyanate is an aromatic isocyanate.

8. A prepolymer as claimed in either of claims 5 or 6 in which the polyol is a propylene oxide adduct.

9. A water hardenable composition for use in a bandage of any of claims 1 to 4 which comprises an isocyanate terminated prepolymer which contains an organic acid anhydride of any of claims 5 to 8 and a catalyst.

10. A composition as claimed in claim 9 in which the catalyst is potassium carbonate.

11. A composition as claimed in claim 9 in which the catalyst is dimorpholino diethylether.

12. A composition as claimed in claim 9 in which the catalyst is bis(2,6-dimethyl morpholino) diethylether.

13. A method of making a water hardenable splinting bandage as claimed in any of claims 1 to 4 which comprises coating or impregnating a flexible fabric carrier, which has apertures of sufficient size to allow water to permeate the bandage and react with the prepolymer, with a mixture of water curable isocyanate terminated prepolymer containing an organic acid anhydride inhibitor and a catalyst.

14. A process for preparing a water curable isocyanate terminated prepolymer as claimed in any of claims 5 to 8 which comprises mixing a polyol with an organic acid anhydride and reacting the polyol with excess isocyanate.

15. A process for preparing a water hardenable composition as claimed in any of claims 9 to 11 which comprises mixing a catalyst with an isocyanate terminated prepolymer which contains an organic acid anhydride

EP 0 328 531 B1

as claimed in any of claims 5 to 8.


## Ansprüche

1. Ein wasserhärtbarer Polyurethan-Schienverband, der ein flexibles Gewebe enthält, das ein Prepolymer mit wasserhärtbaren Isocyanat-Endgruppen aufweist und einen Katalysator hierfür, dadurch gekennzeichnet, dass dem Prepolymer ein Säureanhydrid beigemengt ist.

2. Ein Verband nach Anspruch 1, in dem das Anhydrid ein zweibasisches Säureanhydrid ist.

3. Ein Verband nach Anspruch 2, in dem das Anhydrid Bernsteinsäureanhydrid ist.

4. Ein Verband nach den Ansprüchen 1-3, in dem das Anhydrid in Teilchenform und mit 0,5 bis 1,5 Gew.-% vorliegt bezogen auf das Prepolymer.

5. Ein wasserhärtbares Prepolymer mit Isocyanatendgruppen zur Verwendung in einem Verband nach den Ansprüchen 1 bis 4, in dem das Prepolymer von einem Polyol und einem Isocyanat abgeleitet ist und ein organisches Säureanhydrid enthält.

6. Ein Prepolymer nach Anspruch 5, in dem das Anhydrid Bernsteinsäureanhydrid ist.

7. Ein Prepolymer nach den Ansprüchen 5 oder 6, in dem das Polyol ein Ethylenoxid-Addukt und das Isocyanat ein aromatisches Isocyanat ist.

8. Ein Prepolymer nach den Ansprüchen 5 oder 6, in dem das Polyol ein Propylenoxid-Addukt ist.

9. Eine wasserhärtbare Zusammensetzung zur Verwendung in einen Verband nach den Ansprüchen 1 bis 4, die ein Prepolymer mit Isocyanat-Endgruppen aufweist, das ein organisches Säureanhydrid nach den Ansprüchen 5 bis 8 und einen Katalysator enthält.

10. Eine Zusammensetzung nach Anspruch 9, in der der Katalysator Kaliumcarbonat ist.

11. Eine Zusammensetzung nach Anspruch 9, in der der Katalysator Dimorpholinodiethylether ist.

12. Eine Zusammensetzung nach Anspruch 9, in der der Katalysator Bis(2,6-dimethylmorpholino)diethylether ist.

13. Verfahren zur Herstellung eines wasserhärtbaren Schienverbands nach den Ansprüchen 1 bis 4, welches das Beschichten oder Imprägnieren einer flexiblen Gewebestruktur betrifft, die Öffnungen ausreichender Grösse besitzt um das Eindringen von Wasser in den Verband und dessen Reaktion mit wasserhärtbarem Prepolymer mit Isocyanatendgruppen zu ermöglichen und einen Katalysator enthält.

14. Verfahren zur Herstellung eines Prepolymers mit wasserhärtbaren Isocyanatendgruppen nach den Ansprüchen 5 bis 8, nach dem ein Polyol mit einem organischen Säureanhdrid gemischt wird und das Polyol mit einem Überschuss an Isocyanat umgesetzt wird.

15. Verfahren zur Herstellung einer wasserlöslichen Zusammensetzung nach den Ansprüchen 9 bis 11, nach dem ein Katalysator mit einem Prepolymer mit Isocyanatendgruppen gemischt wird, das ein organisches Säureanhydrid nach den Ansprüchen nach den Ansprüchen 5 bis 8 enthält.


## Revendications

1. Bandage pour faire des plâtres à base de polyuréthane pouvant durcir à l'eau comprenant un tissu flexible portant un prépolymère à terminaison isocyanate pouvant être réticulé à l'eau et un catalyseur pour celui-ci, caractérisé en ce que le prépolymère est en mélange avec un anhydride d'acide organique.

2. Bandage suivant la revendication 1, caractérisé en ce que l'anhydride est un anhydride de di-acide aliphatique.

3. Bandage suivant la revendication 2, caractérisé en ce que l'anhydride est l'anhydride succinique.

4. Bandage suivant l'une quelconque des revendications 1 à 3, caractérisé en ce que l'anhydride est sous forme particulaire et est présent à raison de 0,5% à 1,5% en poids du prépolymère.

5. Prépolymère à terminaison isocyanate pouvant être réticulé à l'eau utile dans un bandage suivant l'une quelconque des revendications 1 à 4, caractérisé en ce que le prépolymère dérive d'un polyol et d'un isocyanate et contient un anhydride d'acide organique.

6. Prépolymère suivant la revendication 5, caractérisé en ce que l'anhydride est l'anhydride succinique.

7. Prépolymère suivant l'une quelconque des revendications 5 ou 6, caractérisé en ce que le polyol est un produit d'addition d'oxyde d'éthylène et que l'isocyanate est un isocyanate aromatique.

8. Prépolymère suivant l'une quelconque des revendications 5 ou 6, caractérisé en ce que le polyol est un produit d'addition de l'oxyde de propylène.

9. Composition pouvant durcir à l'eau utile dans un bandage suivant l'une quelconque des revendications 1 à 4, caractérisée en ce qu'elle comprend un prépolymère à terminaison isocyanate qui contient un anhydride

9

d'acide organique suivant l'une quelconque des revendications 5 à 8 et un catalyseur.

10. Composition suivant la revendication 9, caractérisée en ce que le catalyseur est le carbonate de potassium.

11. Composition suivant la revendication 9, caractérisée en ce que le catalyseur est le dimorpholinodiéthyléther.

12. Composition suivant la revendication 9, caractérisée en ce que le catalyseur est le bis(2,6-diméthylmorpholino)diéthyléther.

13. Procédé de fabrication d'un bandage pour faire des plâtres pouvant durcir à l'eau suivant l'une quelconque des revendications 1 à 4, caractérisé en ce qu'il comprend l'enduction ou l'imprégnation d'un support de tissu flexible qui présente des ouvertures de dimension suffisante pour permettre à l'eau d'imprégner le bandage et de réagir avec le prépolymère, avec un prépolymère à terminaison isocyanate pouvant être réticulé à l'eau contenant un inhibiteur de type anhydride d'acide organique et un catalyseur.

14. Procédé pour préparer un prépolymère à terminaison isocyanate pouvant être réticulé à l'eau suivant l'une quelconque des revendications 5 à 8, caractérisé en ce qu'il comprend le mélange d'un polyol avec un anhydride d'acide organique et la réaction du polyol avec de l'isocyanate en excès.

15. Procédé pour préparer une composition pouvant durcir à l'eau suivant l'une quelconque des revendications 9 à 11, caractérisé en ce qu'il comprend le mélange d'un catalyseur avec un prépolymère à terminaison isocyanate qui contient un anhydride d'acide organique suivant l'une quelconque des revendications 5 à 8.